# EUROPEAN PATENT APPLICATION

(11) **EP 4 389 276 A1**
(43) Date of publication of application: **26.06.2024**
(21) Application number: 22306958.4
(22) Date of filing: 20.12.2022
(51) Int. Cl.: B01J 13/14, A01N 25/28, A23P 10/30, A61K 8/11, A61K 9/50, B01J 13/22, C09B 67/02, C11D 3/50, F28D 20/02

(54) **A METHOD OF MAKING MICROCAPSULES COMPRISING A POLYMERIZED CORE AND THE USE OF THE SAME**

(71) Applicant: Calyxia, 94380 Bonneuil-sur-Marne (FR)
(72) Inventor: MICHEL, Lisa, 94380 Bonneuil-sur-Marne (FR); KOCKENPO, Laure, 94380 Bonneuil-sur-Marne (FR); SADAOUI ROUSSELLE, Alicia, 94380 Bonneuil-sur-Marne (FR); BLANGEARD, Quentin, 94380 Bonneuil-sur-Marne (FR); MAINGRET, Valentin, 94380 Bonneuil-sur-Marne (FR)
(74) Representative: Icosa

(57) **Abstract**

The present invention relates to a solid microcapsule comprising a shell and a core, the shell comprising a crosslinked polymer P1, and the core being inside the shell and comprising at least one polymer P2 crosslinked in three dimensions and entrapping at least one active agent.

## Description

### FIELD OF INVENTION

The present invention relates to a solid microcapsule comprising a shell and a core, the shell comprising a crosslinked polymer P1, and the core being inside the shell and comprising a polymer P2 crosslinked in three dimensions and entrapping at least one active agent. Polymer P2 is different from polymer P1 and is selected from the group consisting of: a poly(ester), a poly(epoxy), a poly(urethane), a (poly)urea, a poly(silicon) and mixtures thereof.

### BACKGROUND OF INVENTION

In the prior art, microcapsules comprising a degradable shell encapsulating an active agent are described. However, many active agents that are of interest for encapsulation are miscible with and/or have an affinity to the polymer that is used as the microcapsule shell, rendering the encapsulation less effective if not impossible. Thus, for the encapsulation of active agents that have a tendency to escape the microcapsule, in particular due to the high volatility of said active agents, the existing microcapsules are not able to achieve the needed retention of the active agent due to the ease of diffusion through the shell.

Therefore, there is a real need to develop microcapsules allowing an efficient retention of active agents which are miscible with and/or have an affinity to the polymer that is used as the microcapsule shell, in particular high volatile active agents.

The present invention resolves this problem by providing microcapsules which are able to achieve the needed retention of the encapsulated active agents, in particular high volatile active agents.

### SUMMARY

The present invention relates to a solid microcapsule comprising:
- a shell comprising a crosslinked polymer P1, and
- a core, said core being inside the shell, wherein said core comprises:
   o at least one polymer P2 crosslinked in three dimensions, said at least one polymer P2, different from polymer P1, being selected from the group consisting of: a poly(ester), a poly(epoxy), a poly(urethane), a poly(urea), a poly(silicon) and mixtures thereof, and
   o at least one active agent entrapped in the at least one crosslinked polymer P2.

Advantageously, a ratio ζ ranges from 0.002 mol/g to 0.015 mol/g, preferably from 0.002 mol/g to 0.01 mol/g. Said ratio ζ is the ratio of the functionality of the at least one type of monomers and/or oligomers from which the polymer P2 is obtained to the molecular weight of the at least one type of monomers and/or oligomers from which the polymer P2 is obtained.

Advantageously, the polymer P2 is obtained from the polymerization of at least one type of monomers and/or oligomers selected from the group consisting of:
- unsaturated monomers bearing at least one function selected in the group consisting of: (meth)acrylate function; primary, secondary or tertiary alkylamine functions; quaternary amine functions; sulfate function; sulfonate function; phosphate function; phosphonate function; carboxylate function; hydroxyl function; halogen function and mixtures thereof,
- oligomers bearing (meth)acrylate functions such as poly(urethane)s, poly(ester)s, poly(urea)s, poly(ether)s and poly(dimethylsiloxane)s,
- and mixtures thereof.

Advantageously, the polymer P2 has a glass transition temperature greater than or equal to 30°C, the glass transition temperature being determined by differential scanning calorimetry analysis, preferably by differential scanning calorimetry analysis using a TA Instruments DSC 25.

Advantageously, the at least one active agent is liquid or solid and the core further comprises at least one oil which surrounds the at least one active agent and which is entrapped in the polymer P2.

Advantageously, the core further comprises at least one gelling agent entrapped in the polymer P2, preferably said gelling agent is selected from the group consisting of: a gelling polymer, a hydrogel and a viscose oil.

Advantageously, the core further comprises an intermediate phase, wherein said intermediate phase is not miscible with the polymer P1. Preferably, said intermediate phase is present between the at least one polymer P2 and the polymer P1. Preferably, said intermediate phase is an aqueous intermediate phase.

In one embodiment, the at least one active agent is solid and the core consists in the polymer P2 and the at least one active agent.

Advantageously, said solid microcapsule further comprises a coating surrounding the shell, said coating comprising a polymer P4 different from polymers P1 and P2, said polymer P4 being preferably selected from the group consisting of: a cationic polymer, an anionic polymer and an amphoteric polymer.

The present invention also relates to a method for preparing solid microcapsules comprising the steps of:
a) adding, in a composition C2, at least one active agent, with stirring, to obtain a mixture M1,
wherein the composition C2 comprises:
   - at least one oil,
   - at least one type of monomers and/or oligomers, each of the at least one type of monomers and/or oligomers bearing, independently from one another, at least one functional group selected from the group consisting of: acrylate, ester, epoxy, urea, silicon and urethane, and
   - optionally at least one initiator and/or at least one surfactant,
a1) optionally, adding the mixture M1 in an aqueous composition comprising at least one type of water-soluble monomers and/or oligomers and/or at least one gelling agent, and optionally a surfactant, to obtain an emulsion (E1),
b) adding, with stirring, the mixture M1 obtained in step a) or the optional emulsion (E1) obtained in step a1) in a composition C3 to obtain an emulsion (E2) comprising either drops of the mixture M1 dispersed in composition C3, or drops of the emulsion (E1) dispersed in composition C3,
wherein the composition C3 comprises at least one photoinitiator and at least one type of monomers and/or oligomers, which are different from the at least one type of monomers and/or oligomers of composition C2, wherein the compositions C3 and C2 are not miscible with each other and wherein the composition C3 and the aqueous composition of step a1) are not miscible with each other,
c) applying a shear to the emulsion (E2) obtained in step b), at a rate of between 100 s⁻¹ and 100,000 s⁻¹,
to obtain an emulsion (E3) comprising controlled-size drops and preferably monodisperse drops of the mixture M1 or the emulsion (E1) dispersed in the composition C3;
d) adding, with stirring, the emulsion (E3) obtained in step c) in a composition C4 to obtain an emulsion (E4) comprising drops of the emulsion (E3) dispersed in composition C4, wherein the compositions C3 and C4 are not miscible with each other,
the viscosity of composition C4 being greater than 10,000 mPa.s at 25° C at a shear rate of 10 s⁻¹ and being less than 10,000 mPa.s at 25° C at a shear rate of between 100 s⁻¹ and 100,000 s⁻¹; and
e) polymerizing of the compositions C2 and C3, to obtain solid microcapsules, dispersed in the composition C4.

Advantageously, the monomers and/or oligomers of composition C2 have a functionality of at least 2, preferably of at least 5, more preferably of at least 10, even more preferably of at least 16.

Advantageously, the at least one type of monomers and/or oligomers of composition C2 is selected from the group consisting of: 1,6-hexanediol diacrylate, 3-methyl-1,5-pentanediyl diacrylate, tripropylene glycol diacrylate, propylidynetrimethyl trimethacrylate, trimethylolpropane triacrylate, 1,10-decanediyl diacrylate, bisphenol A epoxy diacrylate, epoxidized soybean oil acrylate, polyester acrylate oligomer, dendritic acrylate, epoxy acrylate oligomer, urethane acrylates, polyester acrylate, epoxy acrylate, amine acrylate, oligoamine acrylate, silicon elastomers and combinations thereof.

Advantageously, the at least one active agent of composition C1 has a solubility in the composition C3 which is equal to or greater than 1 % w/w.

Advantageously, the mixture M1 comprises:
- from 45% to 99%, preferably from 50% to 70%, more preferably about 70%, of the at least one active agent,
- from 0.5% to 5%, preferably from 1.5% to 4%, more preferably from 1.5% to 3%, of the at least one initiator,
- from 0.5% to 50%, preferably from 5% to 15%, more preferably from 10% to 13%, of the at least one type of monomers and/or oligomers,
the percentages being expressed by weight relative to the total weight of the mixture M1.

Advantageously, the mixture M1 comprises:
- from 10% to 40%, preferably from 20% to 40%, more preferably about 30%, of the at least one active agent,
- from 0.5% to 5%, preferably from 1.5% to 4%, more preferably from 1.5% to 3%, of the at least one initiator,
- from 55% to 89.5%, preferably from 55% to 80%, more preferably from 55% to 65%, of the at least one type of monomers and/or oligomers,
the percentages being expressed by weight relative to the total weight of the mixture M1.

Advantageously, the at least one initiator of the composition C2 and/or the at least one photoinitiator of the composition C3 is selected from the group consisting of: 2-hydroxy-2-methylpropiophenone, phenyl(2,4,6-trimethylbenzoyl)phosphinate ethyl, bis(2,4,6-trimethylbenzoyl)phenylphosphine oxide and combinations thereof.

Advantageously, said method is for preparing solid microcapsules according to the present invention.

### DEFINITIONS

In the present invention, the following terms have the following meanings:

**"About",** before a figure or number, refers to plus or minus 10% of the face value of that figure or number. In one embodiment, "about", before a figure or number, refers to plus or minus 5% of the face value of that figure or number.

**"Active agent"** or **"active ingredient"** refers to a substance that has an effect, in particular in a field selected from the group consisting of therapeutic, cosmetic, agriculture, home care, personal care, chemical, paint, fuel, lubricant, bitumen, and drilling sludges or muds. The agent may be a chemical or a biological substance. Preferably, the active agent is a chemical substance.

Advantageously, the active agent is selected from the group consisting of:
- a crosslinker, hardener, curing agent (such as a urea derivative), curing agent accelerator (such as amide, amine and imidazole derivatives), organic or metallic catalyst (such as an organometallic or inorganometallic complex of platinum, palladium, titanium, molybdenum, copper, zinc or salts of metallic complexes dissolved in polyethylene glycol or polyethylene glycol derivatives) used to polymerize polymer, elastomer, rubber, paint, adhesive, sealant, mortar, varnish or coating formulations;
- a colorant or pigment for use in elastomer, paint, coating, adhesive, sealant, mortar, or paper formulations;
- a fragrance (as defined by the International Fragrance Association (IFRA) list of molecules available at www.ifraorg.org) for use in detergents such as laundry detergents, home care products, cosmetics and personal care products, textiles, paints, coatings;
- a flavouring, a vitamin, an amino acid, a protein, a lipid, a probiotic, an antioxidant, a pH corrector, a preservative for food compounds and animal feed;
- a plant extract or an essential oil derivative such as propolis extract, menthol derivatives or poppy petal extract for personal care formulations;
- a softener, a moisturizer, a conditioner for detergents, laundry, cosmetics and personal care products. In this respect, the active ingredients that can be used are, for example, listed in patents US 6 335 315 and US 5 877 145;
- an anti-color alteration agent (such as an ammonium derivative), an anti-foaming agent (such as an alcohol ethoxylate, an alkylbenzene sulfonate, a polyethylene ethoxylate, an alkyl ethoxysulfate or an alkyl sulfate) for detergents and laundry and home care products;
- a brightening agent, also known as a color activator (such as a stilbene derivative, a coumarin derivative, a pyrazoline derivative, a benzoxazole derivative or a naphthalimide derivative) for use in detergents, laundry products, cosmetics and personal care products;
- a biologically active compound such as an enzyme, a vitamin, a protein, a plant extract, an emollient agent, a disinfectant agent, an antibacterial agent, an anti-UV agent, a drug intended for cosmetic and personal care products, and textiles. Among these biologically active compounds we can mention : vitamins A, B, C, D and E, para aminobenzoic acid, alpha hydroxy acids (such as glycolic acid, lactic acid, malic acid, tartaric acid or citric acid), camphor, ceramides, polyphenols (such as flavonoids, phenolic acid, ellagic acid, tocopherol, ubiquinol), hydroquinone, hyaluronic acid, isopropyl isostearate, isopropyl palmitate, oxybenzone, panthenol, proline, retinol, retinyl palmitate, salicylic acid, sorbic acid, sorbitol, triclosan, tyrosine;
- a disinfectant, an antibacterial agent, an anti-UV agent, for paints and coatings;
- a fertilizer, herbicide, insecticide, pesticide, fungicide, repellent or disinfectant for agrochemicals;
- a flame retardant (such as a brominated polyol like tetrabromobisphenol A, a halogenated or non-halogenated organophosphorus compound, a chlorine compound, a perfluorinated compound, an ammonium polyphosphate, an aluminum trihydrate, an antimony oxide, a zinc borate, a red phosphorus, a melamine, or a magnesium dihydroxide) for use in plastics, coatings, paints and textiles;
- a photonic crystal or photochromophore for use in paints, coatings and polymeric materials forming curved and flexible displays; and
- a Phase Change Materials (PCM) capable of absorbing or releasing heat when undergoing a phase change, intended for energy storage. Examples of PCMs include molten aluminum phosphate, ammonium carbonate, ammonium chloride, cesium carbonate, cesium sulfate, calcium citrate, calcium chloride, calcium hydroxide, calcium oxide, calcium phosphate, calcium saccharate, calcium sulfate, cerium phosphate, iron phosphate, lithium carbonate, lithium sulfate, magnesium chloride, magnesium sulfate, manganese chloride, manganese nitrate, manganese sulfate, potassium acetate, potassium carbonate, potassium chloride, potassium phosphate, rubidium carbonate, rubidium sulfate, disodium tetraborate, sodium acetate, sodium bicarbonate, sodium bisulfate, sodium citrate, sodium chloride, sodium hydroxide, sodium nitrate, sodium percarbonate, sodium persulfate sodium phosphate, sodium propionate, sodium selenite, sodium silicate, sodium sulfate, sodium tellurate, sodium thiosulfate, strontium hydrophosphate, zinc acetate, zinc chloride, sodium thiosulfate, paraffinic hydrocarbon waxes, and polyethylene glycols.

Advantageously, the active agent is hydrophilic. More advantageously, the active agent is selected from the group consisting of:
- a hydrophilic crosslinker, hardener, curing agent accelerator (such as amide, amine and imidazole derivatives), or a hydrophile metallic catalyst such as salts of metallic complexes dissolved in polyethylene glycol or polyethylene glycol derivatives used to polymerize polymer, elastomer, rubber, paint, adhesive, sealant, mortar, varnish or coating formulations;
- a hydrophilic colorant or pigment for use in elastomer, paint, coating, adhesive, sealant, mortar, or paper formulations;
- a hydrophilic flavouring, vitamin, amino acid, protein, probiotic, antioxidant, pH corrector, preservative for food compounds and animal feed;
- a hydrophilic plant extract or essential oil derivative such as propolis extract, menthol derivatives or poppy petal extract for personal care formulations;
- a hydrophilic softener, conditioner for detergents, laundry, cosmetics and personal care products selected from amine derivatives;
- a hydrophilic biologically active compound selected from hydrophile enzymes, vitamins, proteins, antibacterial agents.
- a hydrophilic fertilizer, herbicide, insecticide, pesticide, fungicide, repellent or disinfectant for agrochemicals;
- a hydrophilic flame retardant such as a chlorine compound or an ammonium polyphosphate, for use in plastics, coatings, paints and textiles;
- a hydrophilic Phase Change Materials (PCM) such as ammonium carbonate, ammonium chloride, calcium chloride, calcium hydroxide, calcium phosphate, calcium saccharate, calcium sulfate, magnesium chloride, magnesium sulfate, manganese chloride, manganese nitrate, manganese sulfate, potassium acetate, potassium carbonate, potassium chloride, potassium phosphate, sodium acetate, sodium bicarbonate, sodium bisulfate, sodium chloride, sodium hydroxide, sodium nitrate, sodium percarbonate, sodium persulfate, sodium phosphate, sodium propionate, sodium sulfate, sodium tellurate, sodium thiosulfate, zinc acetate, zinc chloride, sodium thiosulfate, and polyethylene glycols.

**"Aqueous phase"** refers to a solution comprising a solvent and optionally at least one solute, wherein said solvent is selected from the group consisting of water, hydrophilic organic solvents and combinations thereof. According to the invention, an aqueous phase and an oil phase are immiscible in each other, which means that the amount by weight of the aqueous phase capable of being solubilized in the oil phase is less than or equal to 5%, preferably less than 1%, more preferably less than 0, 5%, even preferably of 0%, based on the total weight of the oil phase, and that the amount (by weight) of the oil phase capable of being solubilized in the aqueous phase is less than or equal to 5%, preferably less than 1%, more preferably less than 0.5%, even preferably of 0%, based on the total weight of the aqueous phase. Advantageously, the immiscibility between the aqueous phase and the oil phase makes it possible to avoid migration of active agent(s) optionally present in the aqueous phase from said aqueous phase to the oil phase.

**"At least"** means "greater than or equal to" and is thus different from "strictly greater than" which does not comprise "equal to".

**"Comprising"** or **"comprise"** is to be construed in an open, inclusive sense, not limited to the features following this term.

**"Consisting of"** or **"consist"** is to be construed in a close, non-inclusive sense, limited to the features following this term.

**"Crosslinkable"** refers to a composition capable of polymerizing (crosslinking) to give a solid material, for example to form the polymerized shell or the polymerized polymer in the core of solid microcapsules according to the invention.

**"Drop"** or **"droplet"** refers to a small liquid particle. **"Single drop"** refers to a drop consisting of a single drop phase. **"Double drop"** refers to a drop comprising two phases in the drop, organized in the form of at least one internal drop (composed of one phase) surrounded by an external drop (composed of the other phase).

**"Emulsion"** refers to a fluid colloidal system in which liquid droplets are dispersed in a liquid. The droplets often exceed the usual limits for colloids in size. A **"simple emulsion"** is denoted by the symbol O/W (or by the term oil-in-water) if the continuous phase is an aqueous solution (=aqueous phase) and by W/O (or by the term water-in-oil) if the continuous phase is an organic liquid (an "oil"). **"Double emulsions"** are more complicated emulsion, such as W/O/W (also named water-in-oil-in-water double emulsion, i.e. aqueous droplets contained within oil droplets dispersed in a continuous aqueous phase). In a W(1)/O/W(2) double emulsion, the internal emulsion refers to the emulsion of the innermost aqueous phase W(1) in the oil phase O; and the external emulsion refers to the emulsion of the oil phase O in the external aqueous phase W(2). **"Triple emulsions"** are more complicated emulsion, such as O/W/O/W (also named oil-in-water-in-oil-in-water triple emulsion, i.e. oil droplets contained within aqueous droplets contained within oil droplets dispersed in a continuous aqueous phase). In a O(1)/W(1)/O(2)/W(2) triple emulsion, the internal emulsion refers to the emulsion of the innermost oil phase O(1) in the aqueous phase W(1); the intermediate emulsion refers to the emulsion of the aqueous phase W(1) in the oil phase O(2); and the external emulsion refers to the emulsion of the oil phase O(2) in the external aqueous phase W(2).

**"From X to Y"** refers to the range of values between X and Y, the limits X and Y being included in said range.

**"Hydrophilic"** or **"hydrophile"** refers to the capacity of a molecular entity to interact with polar solvents, in particular with water.

**"Immiscibility"** between two phases (named phase 1 and phase 2) means that the amount by weight of the phase 1 capable of being solubilized in the phase 2 is less than or equal to 5%, preferably less than 1%, more preferably less than 0, 5%, more preferably of 0%, based on the total weight of the phase 2, and that the amount (by weight) of the phase 2 capable of being solubilized in the phase 1 is less than or equal to 5%, preferably less than 1%, more preferably less than 0.5%, even preferably of 0%, based on the total weight of the phase 1.

**"Initiator"** refers to any substance introduced in the oil phase in order to bring about a polymerization reaction when said initiator is activated by a source of energy, such as mechanical stirring, heat or light radiation. **"Photoinitiator"** refers to any substance introduced in the oil phase in order to bring about a polymerization reaction when said photoinitiator is activated by a light radiation.

**"Mean diameter"** refers to the Dn₅₀ diameter. Dn₅₀ is the median of the number averaged size distribution of the droplets or (micro)capsules. The size distribution of the droplets or (micro)capsules, and thus the mean diameter of the droplets or (micro)capsules, may be measured by methods well known to the skilled person in the art, e.g. by a light scattering technique (for example using a Mastersizer 3000 equipped with a hydro SV measuring cell), or by image analysis of optical microscopy pictures, or by image analysis of electronic microscopy pictures (SEM - Scanning electron microscopy).

**"Monomer"** refers to a molecule which can undergo polymerization thereby contributing constitutional units to the essential structure of a macromolecule. **"Oligomer"** refers to a macromolecule comprising a repetition of monomers, preferably a repetition of less than 10 monomers.

**"Oil phase"** refers to a phase not miscible with an aqueous phase, which means that the amount by weight of an aqueous phase capable of being solubilized in the oil phase is less than or equal to 5%, preferably less than 1%, more preferably less than 0, 5%, even preferably of 0%, based on the total weight of the oil phase, and that the amount (by weight) of the oil phase capable of being solubilized in an aqueous phase is less than or equal to 5%, preferably less than 1%, more preferably less than 0.5%, even preferably of 0%, based on the total weight of the aqueous phase. The oil phase does not necessarily contain oil. Advantageously, the immiscibility between the aqueous phase and the oil phase makes it possible to avoid migration of active agent(s) optionally present in the oil phase from said oil phase to the aqueous phase.

**"Polymerization"** refers to the process of converting a mixture of monomers and/or oligomers into a polymer.

**"Powder"** refers to a set of dry and more or less fine solid particles. **"Particle"** refers to a very small part of a material element. Advantageously, the mean diameter of the particles ranges from 1 nm to 10 µm, preferably from 1 nm to 1 µm, preferably from 10 nm to 10 µm, more preferably from 10 nm to 500 nm, even more preferably from 1 nm to 100 nm. **"Dry"** refers to a material, composition or substance comprising less than 1% w/w of water, preferably less than 0.5% w/w of water, more preferably less than 0.01% w/w of water.

**"Shear rate Y"** refers to the velocity gradient in a flowing fluid. The **"shear stress σ"** applied to an emulsion drop is defined as the tangential force per unit drop area resulting from the macroscopic shear applied to the emulsion during its agitation. The shear stress σ (expressed in Pa), the viscosity η (expressed in Pa.s) and the shear rate Y (expressed in s⁻¹) applied to an emulsion during its agitation are related by the following equation: σ = ηY.

**"Viscosity"** or **"dynamic viscosity"** refers, for a laminar flow of a fluid, to the ratio of the shear stress to the velocity gradient perpendicular to the plane of shear. Viscosity may be measured by methods well-known to a skilled person in the art. Unless otherwise indicated, viscosity, in the present application, is measured by any viscosity measurement method well known to person skilled in the art. In particular, viscosity may be measured using a TA Instruments AR-G2 rheometer equipped with a 3 cm diameter, 2-degree angle cone with a plate geometry and a temperature control cell set at 25°C, the viscosity value being read at a shear rate ranging from 0.1 s⁻¹ to 1000 s⁻¹, preferably at a shear rate equal to 10 s⁻¹.

**"Volatile active agent"** refers to a compound capable of evaporating in less than one hour, at ambient temperature (25° C) and atmospheric pressure (760 mmHg). A volatile compound according to the invention is therefore liquid at ambient temperature, in particular having a non-zero vapor pressure, at ambient temperature and atmospheric pressure, in particular having a vapor pressure ranging from 0.13 Pa to 40 000 Pa (0.001 mmHg to 300 mmHg), and preferably from 1.3 Pa to 13 000 Pa (0.01 mmHg to 100 mmHg), and preferably ranging from 1.3 Pa to 1300 Pa (0.01 mmHg to 10 mmHg). The evaporation rate of a volatile compound according to the invention may be evaluated, in particular, by means of the protocol described in the international application WO2006/013413.

### DETAILED DESCRIPTION

### Solid microcapsule

This invention relates to a solid microcapsule comprising:
- a shell comprising a crosslinked polymer P1, and
- a core, said core being inside the shell, wherein said core comprises:
   o at least one polymer P2 crosslinked in three dimensions, said at least one polymer P2, different from polymer P1, being selected from the group consisting of: a poly(ester), a poly(epoxy), a poly(urethane), a (poly)urea, a poly(silicon) and mixtures thereof, and
   o at least one active agent entrapped in the at least one polymer P2.

Advantageously, the mean diameter of the solid microcapsules ranges from 1 µm to 70 µm.

The inventors of the present invention have surprisingly discovered that a solid microcapsule according to the invention allows a good retention of active agents in the core, even high volatile active agents, the active agent being either solid (and thus in an oil entrapped in polymer(s) P2 or directly entrapped in polymer(s) P2) or liquid (and thus present in an oil entrapped in polymer(s) P2, an intermediate phase being optionally present in the core when the active agent is hydrophobic).

### Polymer P1 (shell)

Advantageously, the polymer P1 is obtained from the polymerization of at least one type of monomers and/or oligomers having a functionality of strictly less than 8, preferably strictly less than 6.

Advantageously, the ratio of the functionality of the oligomer to its molecular weight, further referred to as "ζ value", or "ζ" or "ratio ζ", ranges from 0.002 mol/g to 0.015 mol/g, preferably from 0.002 mol/g to 0.01 mol/g.

Advantageously, the polymer P1 is obtained from the polymerization of at least one type of monomers and/or oligomers selected from monomers and oligomers comprising at least one reactive function selected from the group consisting of acrylate, methacrylate, vinyl ether, N-vinyl ether, vinyl ester, mercaptoester, thiolene, maleate, epoxy, siloxane, amine, lactone, phosphate, carboxylate, oxetane, urethane, isocyanate and peroxide moieties. More advantageously, the polymer P1 is obtained from the polymerization of at least one type of monomers and/or oligomers selected from the group consisting of: aliphatic or aromatic esters or poly(ester)s, urethanes or poly(urethane)s, anhydrides or poly(anhydride)s, saccharides or poly(saccharide)s, ethers or poly(ether)s, amides or poly(amide)s, and carbonates or poly(carbonate)s, wherein the monomers or oligomers further include at least one reactive moiety selected from the group consisting of acrylate, methacrylate, vinyl ether, N-vinyl ether, vinyl ester, mercaptoester, thiolene, maleate, epoxy, siloxane, amine, lactone, phosphate, carboxylate, oxetane, urethane, isocyanate and peroxide moieties.

Advantageously, the polymer P1 is selected from the group consisting of: poly(ether)s, poly(epoxide)s, poly(ester)s, poly(urethane)s, poly(urea)s, poly(ethylene glycol)s, poly(propylene glycol)s, poly(amide)s, poly(acetal)s, poly(imide)s, poly(olefin)s, poly(sulfide)s and poly(dimethylsiloxane)s. More advantageously, the polymer P1 may be selected from the group consisting of: poly(ester)s, poly(epoxide)s, and poly(urethane)s. Even more advantageously, said polymer P1 further carries at least one reactive functionality selected from the group consisting of acrylate, methacrylate, vinyl ether, N-vinyl ether, mercaptoester, thiolene, siloxane, epoxy, oxetane, urethane, isocyanate, and peroxide functionality. For example, the polymer P1 may be selected from the group consisting of: poly(2-(1-naphthyloxy)-ethyl acrylate), poly(2-(2-naphthyloxy)-ethyl acrylate), poly(2-(2-naphthyloxy)-ethyl methacrylate), polysorbitol dimethacrylate, polyacrylamide, poly((2-(1-naphthyloxy) ethanol), poly(2-(2-naphthyloxy) ethanol), poly(1-chloro-2,3-epoxypropane), poly(n-butyl isocyanate), poly(N- vinyl carbazole), poly(N-vinyl pyrrolidone), poly(p-benzamide), poly(p-chlorostyrene), poly(p-methyl styrene), poly(p-phenylene oxide), poly(p-phenylene sulfide), poly(N-(methacryloxyethyl)succinimide), polybenzimidazole, polybutadiene, polybutylene terephthalate, polychloral, polychloro trifluoro ethylene polyether imide, polyether ketone, polyether sulfone, polyhydridosilsesquioxane, poly(m-phenylene isophthalamide), poly(methyl 2-acrylamido-2-methoxyaceate), poly(2-acrylamido-2-methylpropanesulfonic acid), poly-mono-butyl maleate, polybutylmethacrylate, poly(N-tert-butylmethacrylamide), poly(N-n-butylmethacrylamide), polycyclohexylmethacrylamide, poly(m-xylenebisacrylamide 2,3-dimethyl-1,3-butadiene, N,N-dimethylmethacrylamide), poly(n-butyl methacrylate), poly(cyclohexyl methacrylate), polyisobutyl methacrylate, poly(4-cyclohexylstyrene), polycyclol acrylate, polycyclol methacrylate, polydiethyl ethoxymethylenemalonate, poly(2,2,2-trifluoroethyl methacrylate), poly(1,1,1-trimethylolpropane trimethacrylate), polymethacrylate, poly(N,N-dimethylaniline, dihydrazide), poly(isophthalic dihydrazine) polyisophthalic acid, polydimethyl benzilketal, epichlorohydrin, poly(ethyl-3,3-diethoxyacrylate), poly(ethyl-3,3-dimethylacrylate), poly(ethyl vinylketone) poly(penten-3-one), polyformaldehyde poly(diallyl acetal), polyfumaronitrile, polyglyceryl propoxy triacrylate, polyglyceryl trimethacrylate, polyglycidoxypropyltrimethoxysilane, polyglycidyl acrylate poly(n-heptyl acrylate), poly(n-heptyl acrylic acid ester), poly(n-heptyl methacrylate), poly(3-hydroxypropionitrile), poly(2-hydroxypropyl acrylate) poly(N-(methacryloxyethyl)phthalimide), poly(1,9-nonanediol diacrylate), poly(1,9-nonanediol dimethacrylate), poly(N-(n-propyl) acrylamide), poly(ortho-phthalic acid), poly(isophthalic acid), poly(1, 4-benzenedicarboxylic acid), poly(1,3-benzenedicarboxylic acid), poly(phthalic acid), poly(mono-2-acryloxyethyl ester), polyterephthalic acid, polyphthalic anhydride, polyethylene glycol diacrylate, polyethylene glycol methacrylate, polyethylene glycol dimethacrylate, poly(isopropyl acrylate), polysorbitol pentaacrylate, polyvinyl bromoacetate, polychloroprene, poly(di-n-hexyl silylene) poly(di-n-propyl siloxane), polydimethyl silylene, polydiphenyl siloxane, polyvinyl propionate, polyvinyl triacetoxysilane, polyvinyl tris-tert-butoxysilane, polyvinyl butyral, polyvinyl alcohol, polyvinyl acetate polyethylene co-vinyl acetate, poly(bisphenol-A polysulfone), poly(1,3-dioxepane), poly(1,3-dioxolane), poly(1,4-phenylene vinylene), poly(2,6-dimethyl-1A-phenylene oxide), poly(4-hydroxybenzoic acid) poly(4-methyl pentene-1), poly(4-vinyl pyridine), polymethylacrylonitrile, polymethylphenylsiloxane, polymethylsilmethylene, polymethylsilsesquioxane, poly(phenylsilsesquioxane), poly(pyromellitimide-1. 4-diphenyl ether), polytetrahydrofuran, polythiophene, poly(trimethylene oxide), polyacrylonitrile, polyether sulfone, polyethylene-co-vinyl acetate, poly(perfluoroethylene propylene), poly(perfluoroalkoxyl alkane and poly(styrene-acrylonitrile).

Advantageously, the polymer P1 may have a molecular weight ranging from 100 g.mol⁻¹ to 10,000 g.mol⁻¹, preferably from 200 g.mol⁻¹ to 10,000 g.mol⁻¹, more preferably from 200 g.mol⁻¹ to 5,000 g.mol⁻¹.

Advantageously, the polymer P1 has a glass temperature greater than or equal to 50°C, for example of 74°C. The glass temperature may be determined by differential scanning calorimetry analysis. In particular, the glass temperature may be determined by differential scanning calorimetry analysis using a TA Instruments DSC 25.

Advantageously, the polymers P1 and P2 are immiscible.

### Polymer P2 (core)

Advantageously, the at least one polymer P2 has a functionality of at least 2.

Advantageously, the functionality of the at least one polymer P2 is greater than the functionality of the polymer P1.

Advantageously, the at least one polymer P2 expands through the core. More advantageously, the at least one polymer P2 forms a polymer network across the entirety of the core through polymerization. Alternatively, but also more advantageously, the polymer network may form along the entirety of the perimeter of the core, thereby preventing diffusion of the volatile component into the shell polymer network comprised of polymer P1.

Advantageously, the at least one polymer P2 is hydrosoluble.

Advantageously, the at least one polymer P2 is selected from the group consisting of: a poly(ester), a poly(epoxy), a poly(urethane), a (poly)urea and mixtures thereof. More advantageously, the at least one polymer P2 is selected from the group consisting of: a poly(ester), a poly(epoxy), a poly(urethane) and mixtures thereof. Even more advantageously, the at least one polymer P2 is selected from the group consisting of: a poly(ester), a poly(epoxy) and mixtures thereof.

Advantageously, the at least one polymer P2 is a poly(ester).

Advantageously, the at least one polymer P2 is a poly(epoxy).

Advantageously, the at least one polymer P2 is a poly(urethane).

Advantageously, the at least one polymer P2 is a poly(urea).

Advantageously, the at least one polymer P2 is obtained from the polymerization of at least one type of monomers and/or oligomers selected from the group consisting of: unsaturated monomers bearing at least one function selected in the group consisting of: (meth)acrylate function; primary, secondary or tertiary alkylamine functions; quaternary amine functions; sulfate function; sulfonate function; phosphate function; phosphonate function; carboxylate function; hydroxyl function; halogen function and mixtures thereof, and oligomers bearing (meth)acrylate functions such as poly(ureathane)s, poly(ester)s, poly(urea)s, poly(ether)s, poly(dimethylsiloxane)s.

Advantageously, the level of polymerization of the at least one polymer P2 is tailored to control the release rate of the at least one active agent entrapped in the at least one polymer P2 to the shell of the solid microcapsule. For example, the level of polymerization of polymer P2 may preferably range from 50% to 100%, more preferably from 70% to 100%, the percentages being a number percentage. The level of polymerization corresponds to the number of acrylate functions that have disappeared after polymerization, compared to the number of total acrylate functions present on all the monomers and/or oligomers before polymerization.

The level of polymerization of the at least one polymer P2 can be determined through Fourier transform Infrared Spectroscopy (FTIR) in the case of UV polymerization.

Using FTIR, the conversion of reactive groups can be determined by the monitoring of the disappearance of one band representative of a functional group, the absorption of IR bands being proportional to the amount of the functional group, therefore the reduction of peak height corresponds to the reduction of the amount of the functional group, further indicating successful polymerization. The standard method of doing this is comparison of the FTIR absorption of the emulsion before and after photopolymerization. For the purpose of the present invention this can be done using the method disclosed in Barszczewska-Rybarek, Materials 2019, 12(24), 4057.

Advantageously, the at least one polymer P2 has a glass temperature greater than or equal to 30°C. This helps to avoid the diffusion of the active agent from the core to the shell and it helps to avoid miscibility between the polymers P1 and P2. In particular, if the core further comprises a gelling agent, a glass temperature of polymer P2 greater than or equal to 30°C contributes to the formation of a gel in the core.

The glass temperatures may be determined by differential scanning calorimetry analysis. In particular, the glass temperatures may be determined by differential scanning calorimetry analysis using a TA Instruments DSC 25.

Advantageously, the at least one polymer P2 has a weight average molar mass greater than those of polymer P1.

Advantageously, the molecular weight of the at least one type of monomers and/or oligomers from which the at least one polymer P2 is obtained is higher than the molecular weight of the at least one type of monomers and/or oligomers from which polymer P1 is obtained. Indeed, this allows minimizing the interactions between the at least one type of monomers and/or oligomers from which polymer P1 and P2 are obtained and maximizing the interactions between the at least one type of monomers and/or oligomers from which the at least one polymer P2 is obtained.

Advantageously, the at least one polymer P2 is obtained from the polymerization of at least one type of monomers and/or oligomers having a functionality of at least 5, preferably of at least 10, more preferably of at least 16. More advantageously, the at least one polymer P2 is obtained from the polymerization of at least one type of monomers and/or oligomers having a functionality of 16.

### Active agent and oil (core)

The at least one active agent may be liquid or solid.

Advantageously, the core of the solid microcapsule according to the invention may further comprise, entrapped in the crosslinked polymer P2, at least one oil.

Advantageously, the at least one active agent is liquid and the core further comprises at least one oil that surrounds the at least one active agent and is entrapped in the crosslinked polymer P2. More advantageously, said at least one oil may be selected from the group consisting of silicone oils, mineral oils, vegetable oils and mixtures thereof. In particular, the silicone oils may be selected from the group consisting of poly(dimethylsiloxane) (PDMS), poly(monomethylsiloxane) (PPMS), poly(dimethylsiloxane) hydroxy terminated (PDMS-OH) and mixtures thereof. In particular, the mineral oils may be paraffin oils. In particular, the vegetable oils may be selected from the group consisting of soybean oil, sunflower oil, jojoba oil, coconut oil, lanolin oil, castor oil, derivatives thereof and mixtures thereof.

Advantageously, the at least one active agent is solid and the core consists in the crosslinked polymer P2 and the at least one active agent.

Advantageously, the at least one active agent is solid and the core further comprises at least one oil that surrounds the at least one active agent and is entrapped in the crosslinked polymer P2. More advantageously, said at least one oil may be selected from the group consisting of silicone oils, mineral oils, vegetable oils and mixtures thereof. In particular, the silicone oils may be selected from the group consisting of poly(dimethylsiloxane) (PDMS), poly(monomethylsiloxane) (PPMS), poly(dimethylsiloxane) hydroxy terminated (PDMS-OH) and mixtures thereof. In particular, the mineral oils may be paraffin oils. In particular, the vegetable oils may be selected from the group consisting of soybean oil, sunflower oil, jojoba oil, coconut oil, lanolin oil, castor oil, derivatives thereof and mixtures thereof.

Advantageously, when the active agent is solid, it is in the form of a powder.

Advantageously, the active agent has an effect in a field selected from the group consisting of: home care, personal care and agriculture.

Advantageously, the at least one active agent is a volatile active agent.

Advantageously, the at least one active agent has a solubility in the crosslinked polymer P1 that is equal to or greater than 1 % w/w.

Advantageously, the core may further comprise at least one surfactant. More advantageously, the surfactant may be selected from the group consisting of: non-ionic surfactants and/or poloxamer surfactants (such as polysorbate 60 or polysorbate 80). Preferably, the at least one surfactant is present when there is an intermediate phase as described below in the core and when said intermediate phase is entangled with the at least one polymer P2 and located within the polymer P1. Alternatively, the core does not comprise surfactant.

### Intermediate phase (core)

Advantageously, the core further comprises an intermediate phase, wherein said intermediate phase is not miscible with the polymer P1. Preferably, said intermediate phase is an aqueous intermediate phase. Advantageously, the intermediate phase may be present between the at least one polymer P2 and the polymer P1; alternatively, the intermediate phase may be entangled with the at least one polymer P2 and located within the polymer P1.

More advantageously, said intermediate phase is present when the at least one active agent is hydrophobic. According to the invention, an active agent is hydrophobic when it is miscible with the at least one polymer P2 and with the polymer P1.

Advantageously, said intermediate phase comprises at least one polymer P3 and/or at least one gelling agent. Preferably, the gelling agent is selected from the group consisting of: a gelling polymer, a hydrogel and a viscose oil. More preferably, said gelling agent is selected from the group consisting of: dextrin palmitate, hydrogenated styrene isoprene copolymer, caprylic/capric triglyceride, polyurethane-79, amide polymer, castor oil/IPDI copolymer, caprylic/capric triglyceride, carboxymethylcellulose, and alginate. Advantageously, the gelling agent may further comprise a fumed silica. Preferably the polymer P3 is selected from the group consisting of: acrylate polymers (such as acrylated polyester formed from hydroxylated amine acrylates or hydroxylated aliphatic urethane acrylates) and combinations thereof. More preferably, polymer P3 is selected from the group consisting of: water-soluble acrylate polymers and combinations thereof.

In one embodiment, the intermediate phase may comprise water, a surfactant or emulsifier such as a polysorbate, one or more polyethylene glycols, a glyceride and optionally a photoinitiator.

Advantageously, when said intermediate phase is entangled with the at least one polymer P2 and located within the polymer P1, said intermediate phase may comprise at least one surfactant.

### Polymer P4 (coating)

Advantageously, said solid microcapsule further comprises a coating on the shell, said coating comprising a polymer P4. Preferably, said polymer P4 is selected from the group consisting of: a cationic polymer, an anionic polymer and an amphoteric polymer.

### Use of the solid microcapsule

The present invention also relates to the use of a solid microcapsule according to the invention as described above in a home care composition, personal care composition or agrochemical composition.

### Composition comprising a solid microcapsule according to the invention

The present invention further relates to a composition comprising at least one solid microcapsule according to the invention as described above.

Advantageously, the composition comprising at least one solid microcapsule according to the invention as described above is selected from the group consisting of an agrochemical composition, a home care composition and a personal care composition.

### Method for manufacturing the solid microcapsule

The present invention further relates to a method for preparing solid microcapsules, comprising the steps of:
a) adding, in a composition C2, at least one active agent, with stirring, to obtain a mixture M1,
wherein the composition C2 comprises:
   - at least one oil,
   - at least one type of monomers and/or oligomers, each of the at least one type of monomers and/or oligomers bearing, independently from one another, at least one functional group selected from the group consisting of: acrylate, ester, epoxy, urea, silicon and urethane, and
   - optionally at least one initiator and/or surfactant,
a1) optionally, adding the mixture M1 in an aqueous composition comprising at least one type of water-soluble monomers and/or oligomers and/or at least one gelling agent, and optionally at least one surfactant, to obtain an emulsion (E1),
b) adding, with stirring, the mixture M1 obtained in step a) or the optional emulsion (E1) obtained in step a1) in a composition C3 to obtain an emulsion (E2) comprising either drops of the mixture M1 dispersed in composition C3 or drops of the emulsion (E1) dispersed in composition C3,
wherein the composition C3 comprises at least one photoinitiator and at least one type of monomers and/or oligomers, which are different from the at least one type of monomers and/or oligomers of composition C2, wherein the compositions C3 and C2 are not miscible with each other and wherein the composition C3 and the aqueous composition of step a1) are not miscible with each other,
c) applying a shear to the emulsion (E2) obtained in step b), at a rate of between 100 s⁻¹ and 100,000 s⁻¹,
to obtain an emulsion (E3) comprising controlled-size and preferably monodisperse drops of the mixture M1 or the emulsion (E1) dispersed in the composition C3;
d) adding, with stirring, the emulsion (E3) obtained in step c) in a composition C4 to obtain an emulsion (E4) comprising drops of the emulsion (E3) dispersed in composition C4, wherein the compositions C3 and C4 are not miscible with each other,
the viscosity of composition C4 being greater than 10,000 mPa.s at 25° C at a shear rate of 10 s⁻¹ and being less than 10,000 mPa.s at 25° C at a shear rate of between 100 s⁻¹ and 100,000 s⁻¹; and
e) polymerizing of the compositions C2 and C3, to obtain solid microcapsules, dispersed in the composition C4.

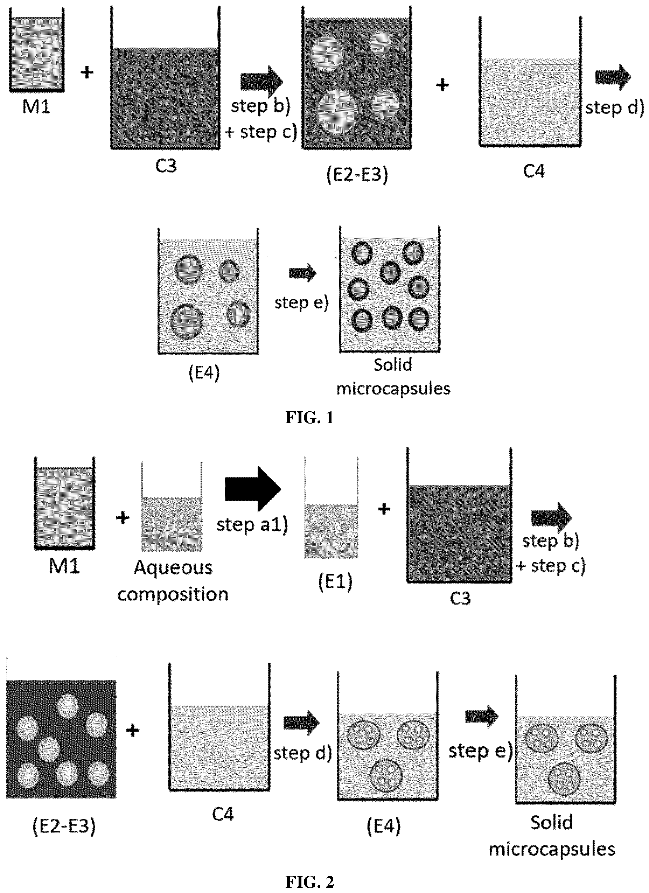

The method according to the invention is illustrated in **Figure 1** and **Figure 2: Fig. 1** illustrates the method according to the invention, wherein step a1) is absent, and **Fig. 2** illustrates the method according to the invention, wherein step a1) is present. As illustrated, when step a1) is present, emulsions (E2) and (E3) are double emulsions and emulsion (E4) is a triple emulsion. When step a1) is absent, emulsions (E2) and (E3) are simple emulsions and emulsion (E4) is a double emulsion.

The at least one active agent may be liquid or solid.

The composition C2 may be a suspension or a solution.

In one embodiment, the at least one active agent is solid and the aqueous composition C2 consists of the at least one active agent, and the at least one type of water-soluble monomers and/or oligomers, each of the at least one type of water-soluble monomers and/or oligomers bearing, independently from one another, at least one functional group selected from the group consisting of: acrylate, ester, epoxy, urea and urethane and potentially at least one photoiniator.

The person skilled in the art understands that it is necessary to have at least two monomers and/or oligomers for each at least one type of monomers and/or oligomers.

Advantageously, the at least one type of monomers and/or oligomers of composition C2 is selected from the group consisting of: 1,6-hexanediol diacrylate, 3-methyl-1,5-pentanediyl diacrylate, tripropylene glycol diacrylate, propylidynetrimethyl trimethacrylate, trimethylolpropane triacrylate, 1,10-decanediyl diacrylate, bisphenol A epoxy diacrylate, epoxidized soybean oil acrylate, polyester acrylate oligomer, dendritic acrylate, epoxy acrylate oligomer, urethane acrylates, polyester acrylate, epoxy acrylate, amine acrylate, oligoamine acrylate, silicon elastomers and combinations thereof.

Advantageously, the at least one active agent has a solubility in the composition C3 which is equal to or greater than 1 %w/w.

Advantageously, the at least one gelling agent is preferably selected from the group consisting of: a gelling polymer, a hydrogel and a viscose oil.

Advantageously, the mixture M1 comprises:
- from 45% to 99%, preferably from 50% to 70%, more preferably about 70%, of the at least one active agent,
- from 0.5% to 5%, preferably from 1.5% to 4%, more preferably from 1.5% to 3%, of the at least one initiator,
- from 0.5% to 50%, preferably from 5% to 15%, more preferably from 10% to 13%, of the at least one type of monomers and/or oligomers,
the percentages being expressed by weight relative to the total weight of the mixture M1.

Advantageously, the mixture M1 comprises:
- from 50% to 80%, preferably from 50% to 70%, more preferably about 70%, of the at least one active agent,
- from 1.5% to 5%, preferably from 1.5% to 4%, more preferably from 1.5% to 3%, of the photo initiators,
- from 1% to 20%, preferably from 5% to 15%, more preferably from 10% to 13%, of the at least one type of monomers and/or oligomers,
the percentages being expressed by weight relative to the total weight of the mixture M1.

Advantageously, the mixture M1 comprises:
- from 10% to 40%, preferably from 20% to 40%, more preferably about 30%, of the at least one active agent,
- from 0.5% to 5%, preferably from 1.5% to 4%, more preferably from 1.5% to 3%, of the at least one initiator,
- from 55% to 89.5%, preferably from 55% to 80%, more preferably from 55% to 65%, of the at least one type of monomers and/or oligomers,
the percentages being expressed by weight relative to the total weight of the mixture M1.

Advantageously, the at least one initiator of the composition C2 is a photoinitiator. More advantageously, the at least one photoinitiator of the composition C2 is selected from the group consisting of: 2-hydroxy-2-methylpropiophenone, phenyl(2,4,6-trimethylbenzoyl)phosphinate ethyl, bis(2,4,6-trimethylbenzoyl)phenylphosphine oxide and combinations thereof.

Advantageously, when step a1) is present and when the resulting intermediate phase is entangled with the at least one polymer P2 and located within the polymer P1, then the composition C2 of step a) comprises at least one surfactant and the aqueous composition of step a1) also comprises at least one surfactant.

Advantageously, the at least one type of water-soluble monomers and/or oligomers at step a1) is selected from the group consisting of: hydroxylated amine acrylates and hydroxylated aliphatic urethane acrylates.

Advantageously, the at least one photoinitiator of the composition C3 is selected from the group consisting of: 2-hydroxy-2-methylpropiophenone, phenyl(2,4,6-trimethylbenzoyl)phosphinate ethyl, bis(2,4,6-trimethylbenzoyl)phenylphosphine oxide and combinations thereof.

Advantageously, the at least one initiator of the composition C2 is at least one photoinitiator, and the at least one photoinitiator of the composition C2 and/or the at least one photoinitiator of the composition C3 is selected from the group consisting of: 2-hydroxy-2-methylpropiophenone, phenyl(2,4,6-trimethylbenzoyl)phosphinate ethyl, bis(2,4,6-trimethylbenzoyl)phenylphosphine oxide and combinations thereof.

Advantageously, at step c), the emulsion (E3) is an emulsion as described in the International patent application published under number WO2022117681, in particular an emulsion having size-controlled droplets whose mean diameter Dn₅₀ is inferior to 100 µm, preferably having a ratio between the viscous modulus (G") and the elastic modulus (G') ranging from 1 to 10. The size control and monodispersity of the drops of emulsion (E3) may advantageously be controlled as described in WO2022117681.

Advantageously, the step e) of polymerizing of composition C2 is carried out by applying UV light. More advantageously, the wavelength of the UV light applied in step e) for polymerizing composition C2 ranges from 300 to 450 nm.

Advantageously, the step e) of polymerizing of composition C3 is carried out by applying UV light. More advantageously, the wavelength of the UV light applied in step e) for polymerizing composition C3 ranges from 300 to 450 nm.

Advantageously, the step e) of polymerizing of the compositions C2 and C3 is carried out by applying UV light. More advantageously, the wavelength of the UV light applied in step e) ranges from 300 to 450 nm.

Advantageously, the step e) of polymerizing of the compositions C2 and C3 is carried out by applying UV light. More advantageously, the same UV wavelength can polymerize both the compositions C2 and C3 simultaneously during step e). Thus, only one step of applying UV length is necessary. Even more advantageously, the same UV wavelength can polymerize both the compositions C2 and C3 simultaneously during step e) and said wavelength of the UV light and ranges from 300 to 450 nm.

The polymerization of composition C2 allows the obtention of the crosslinked polymer of the core of the solid microcapsules, and the polymerization of composition C3 allows the obtention of the crosslinked polymer of the shell of the solid microcapsules.

Advantageously, said method is for preparing solid microcapsules according to the invention as described above. The polymerized composition C2 corresponds to polymer P2 and the polymerized composition C3 corresponds to polymer P1 in the solid microcapsules according to the invention as described above.

### EXAMPLES

The present invention is further illustrated by the following example.

### Example 1: Manufacture of a solid microcapsule according to the invention without an intermediate phase

The following components were used:
- for the core:
   o SR351 trimethylolpropane triacrylate in an amount of 10% w/w relative to the total mass of the composition of the core,
   o active agent (fragrance) in an amount of 70% w/w relative to the total mass of the composition of the core,
   o Massocare DO N (oleic acid, specifically isodecyl oleate) in an amount of 17% w/w relative to the total mass of the composition of the core,
   o TPOL (ethyl phenyl(2,4,6-trimethylbenzoyl)phosphinate)) (commercial name Genocure@ TPOL) in an amount of 3% w/w relative to the total mass of the composition of the core,
- for the shell:
   o CN104D80 (epoxy acrylate) in an amount of 75% w/w relative to the total mass of the composition of the shell,
   o SR 238 (1,6-hexanediol diacrylate) in an amount of 20% w/w relative to the total mass of the composition of the shell,
   o TPOL (ethyl phenyl(2,4,6-trimethylbenzoyl)phosphinate)) (commercial name Genocure@ TPOL) in an amount of 5% w/w relative to the total mass of the composition of the shell,
- for the continuous phase:
   o cellulose derivative in an amount of 8% w/w relative to the total mass of the composition of the continuous phase,
   o water in an amount of 92% w/w relative to the total mass of the composition of the continuous phase.

The components of the core were added in a container and the obtained mixture M was stirred. The components of the shell were added in another container and the obtained mixture M2 was stirred. Then, mixture M was added in the mixture M2 and the global mixture M3 was stirred at a speed of 30 rpm and then at a speed of 70 rpm to obtain a single emulsion N1 containing 60% w/w of the mixture M and 40% w/w of the mixture M2.

The components of the continuous phase were added in another container and the obtained mixture M4 was stirred. Next, the emulsion N1 was added in the mixture M4 and the global mixture M5 was stirred at a speed of 2000 rpm during 1 minute to obtain a double emulsion N2 containing 10% w/w of the emulsion N1 and 90% w/w of the mixture M4.

Finally, the double emulsion N2 was irradiated with UV light at a wavelength of 405 nm during 1 minute and size-controlled capsules according to the invention were obtained, allowing the encapsulation of the fragrance.

### Example 1a: Manufacture of a solid microcapsule according to the invention with an intermediate phase

The following components were used:
- phase A1 for the core (for the intermediate phase entangled in the polymer P2):
   o water in an amount of 82.5% w/w relative to the total mass of the phase A1,
   o surfactant in an amount of 4.00% w/w relative to the total mass of the phase A1,
   o polyethylene glycol diacrylate in an amount of 10.0% w/w relative to the total mass of the phase A1,
   o TPOL (ethyl phenyl(2,4,6-trimethylbenzoyl)phosphinate)) (commercial name Genocure@ TPOL) in an amount of 0.50% w/w relative to the total mass of the phase A1, and
   o Acusol^{®} 880 (containing propylene glycol and water) in an amount of 3.00% w/w relative to the total mass of the phase A1,
- phase A2 for the core (for polymer P2 and active agent):
   o active agent (perfum) in an amount of 68.4% w/w relative to the total mass of the phase A2,
   o Massocare DO N (containing oleic acid, specifically isodecyl oleate) in an amount of 16.6% w/w relative to the total mass of the phase A2,
   o surfactant in an amount of 2.00% w/w relative to the total mass of the phase A2, and
   o SR351 trimethylolpropane triacrylate in an amount of 10.0% w/w relative to the total mass of the phase A2,
   o TPOL (ethyl phenyl(2,4,6-trimethylbenzoyl)phosphinate)) (commercial name Genocure@ TPOL) in an amount of 3.00% w/w relative to the total mass of the phase A2,
- for the shell:
   o CN104D80 (epoxy acrylate) in an amount of 75% w/w relative to the total mass of the composition of the shell,
   o SR 238 (1,6-hexanediol diacrylate) in an amount of 20% w/w relative to the total mass of the composition of the shell,
   o TPOL (ethyl phenyl(2,4,6-trimethylbenzoyl)phosphinate)) (commercial name Genocure@ TPOL) in an amount of 5% w/w relative to the total mass of the composition of the shell,
- for the continuous phase:
   o cellulose derivative in an amount of 8% w/w relative to the total mass of the composition of the continuous phase,
   o water in an amount of 92% w/w relative to the total mass of the composition of the continuous phase.

The components of the phase A2 were added in a container and the obtained mixture M10 was stirred. The components of the phase A1 were added in a second container and the obtained mixture M11 was stirred. The mixture M10 was added in the mixture M11 and the resulting mixture M12 was stirred at a speed of 30 rpm and then at a speed of 70 rpm to obtain a single emulsion E10 containing 50% w/w of the mixture M10 and 50% w/w of the mixture M11. The components of the shell were added in a third container and the obtained mixture M13 was stirred. Then, emulsion E10 was added in the mixture M13 and the resulting mixture M14 was stirred at a speed of 30 rpm and then at a speed of 70 rpm to obtain a double emulsion E11 containing 50% w/w of the mixture M13 and 50% w/w of the emulsion E10.

The components of the continuous phase were added in another container and the obtained mixture M15 was stirred. Next, the emulsion E11 was added in the mixture M15 and the global mixture M16 was stirred at a speed of 2000 rpm during 1 minute to obtain a triple emulsion E12 containing 10% w/w of the emulsion E11 and 90% w/w of the mixture M15.

Finally, the triple emulsion E12 was irradiated with UV light at a wavelength of 405 nm during 1 minute and size-controlled capsules according to the invention were obtained, allowing the encapsulation of the parfum. The industrial processing conditions were as described in European patent application EP22315247.1.

### Example 2: Measure of the leakage improvement of solid microcapsules according to the invention

Three solid microcapsules were prepared:
- solid microcapsule number 1: the core consisted of: 15% w/w of a carrier oil (polyol ester), 70% w/w of an active agent and 15% w/w of a cross-linked polyester, said cross-linked polyester being obtained from the polymerization of monomers and oligomers having a functionality of 2 and a molecular weight of 950 g/mol;
- solid microcapsule number 2: the core consisted of: 15% w/w of a carrier oil (polyol ester), 70% w/w of an active agent and 15% w/w of a cross-linked hydroxyl bearing polyacrylate, said cross-linked hydroxyl bearing polyacrylate being obtained from the polymerization of monomers and oligomers having a functionality of 3 and a molecular weight of 296 g/mol;
- solid microcapsule number 3: the core consisted of: 15% w/w of a carrier oil (polyol ester), 70% w/w of an active agent and 15% w/w of a cross-linked hydroxyl bearing polyacrylate, said cross-linked hydroxyl bearing polyacrylate being obtained from the polymerization of monomers and oligomers having a functionality of 5 and a molecular weight of 525 g/mol.

For the three solid microcapsules, the shell comprised 11% w/w of sodium alginate and 89% w/w of deionized water, the percentages being expressed relative to the total weight of the composition of the shell.

The leakage of the active agent from the three solid microcapsules were determined by the following method: the microcapsules were tested in a model base containing 8% w/w of hydrogenated tallow ester quart (Tetranyl AT7590, Kao Corporation), qsp 100% w/w of water (viscosity of the base: 31 cP at 25°C measured by Brookfield LV1 at 50 rpm). The microcapsules were introduced in said base at a quantity of 0.3% of Active Oil Equivalent (AE) and left for 24 h. Leakage of the active agent was determined through solvent extraction and GC-FID analyses.

AE (active oil equivalent) is defined as the quantity of active agent, which is determined by the weight percentage of the capsule that comprises the active, and the weight percentage of the solution that comprises capsules. Therefore, by way of example, to achieve a 0.3% AE, a solution containing capsules, the capsules containing 50% w/w of active composition relative to the total weight of the capsules, will require 0.6% w/w of the solution to be microcapsules.

The relationship between the active agent retention (leakage improvement) and the ζ value has been determined. For the solid microcapsule number 1, ζ = 2.105×10⁻³ mol/g (with a standard deviation of 5%). For the solid microcapsule number 2, ζ = 10.13×10⁻³ mol/g (with a standard deviation of 5%). For the solid microcapsule number 3, ζ = 9.524×10⁻³ mol/g (with a standard deviation of 5%). For the solid microcapsule number 2, the leakage improvement was of 22.86% compared with the leakage of the solid microcapsule number 1. For the solid microcapsule number 3, the leakage improvement was of 32.86% compared with the leakage of the solid microcapsule number 1.

Thus, the microcapsules according to the invention are able to achieve the retention of encapsulated active agents. In addition, a ζ value of about 10×10⁻³ mol/g allows a better active agent retention than a smaller ζ value of about 2.0×10⁻³ mol/g.

## Claims

1. A solid microcapsule comprising:
- a shell comprising a crosslinked polymer P1, and
- a core, said core being inside the shell, wherein said core comprises:
o at least one polymer P2 crosslinked in three dimensions, said at least one polymer P2, different from polymer P1, being selected from the group consisting of: a poly(ester), a poly(epoxy), a poly(urethane), a poly(urea), a poly(silicon) and mixtures thereof, and
o at least one active agent entrapped in the crosslinked polymer P2.

2. The solid microcapsule according to claim **1,** wherein a ratio ζ of the functionality of the at least one type of monomers and/or oligomers from which the polymer P2 is obtained to the molecular weight of the at least one type of monomers and/or oligomers from which the polymer P2 is obtained ranges from 0.002 mol/g to 0.015 mol/g, preferably from 0.002 mol/g to 0.01 mol/g.

3. The solid microcapsule according to any one of claims **1** to **2,** wherein the polymer P2 is obtained from the polymerization of at least one type of monomers and/or oligomers selected from the group consisting of: unsaturated monomers bearing at least one function selected in the group consisting of: (meth)acrylate function; primary, secondary or tertiary alkylamine functions; quaternary amine functions; sulfate function; sulfonate function; phosphate function; phosphonate function; carboxylate function; hydroxyl function; halogen function and mixtures thereof, and
oligomers bearing (meth)acrylate functions such as polyurethanes, polyesters, polyureas, polyethers, polydimethylsiloxanes.

4. The solid microcapsule according to any one of claims **1** to **3,** wherein the polymer P2 has a glass transition temperature greater than or equal to 30°C, the glass transition temperature being determined by differential scanning calorimetry analysis, preferably by differential scanning calorimetry analysis using a TA Instruments DSC 25.

5. The solid microcapsule according to any one of claims **1** to **4,** wherein the at least one active agent is liquid or solid and the core further comprises at least one oil that surrounds the at least one active agent and is entrapped in the polymer P2.

6. The solid microcapsule according to any one of claims **1** to **5,** wherein the core further comprises at least one gelling agent entrapped in the polymer P2, preferably said gelling agent is selected from the group consisting of: a gelling polymer, a hydrogel and a viscose oil.

7. The solid microcapsule according to any one of claims **1** to **6,** wherein the core further comprises an intermediate phase, wherein said intermediate phase is not miscible with the polymer P1, preferably said intermediate phase is an aqueous intermediate phase, more preferably said intermediate phase is an aqueous intermediate phase present between the at least one polymer P2 and the polymer P1.

8. The solid microcapsule according to any one of claims **1** to **4,** wherein the at least one active agent is solid and the core consists in the polymer P2 and the at least one active agent.

9. The solid microcapsule according to any one of claims **1** to **8,** wherein said solid microcapsule further comprises a coating on the shell, said coating comprising a polymer P4 different from polymers P1 and P2, said polymer P4 being preferably selected from the group consisting of: a cationic polymer, an anionic polymer and an amphoteric polymer.

10. A method for preparing solid microcapsules, comprising the steps of:
a) adding, in a composition C2, at least one active agent, with stirring, to obtain a mixture M1,
wherein the composition C2 comprises:
- at least one oil,
- at least one type of monomers and/or oligomers, each of the at least one type of monomers and/or oligomers bearing, independently from one another, at least one functional group selected from the group consisting of: acrylate, ester, epoxy, urea, silicon and urethane, and
- optionally at least one initiator and/or surfactant,
a1) optionally, adding the mixture M1 in an aqueous composition comprising at least one type of water-soluble monomers and/or oligomers and/or at least one gelling agent, and optionally a surfactant, to obtain an emulsion (E1),
b) adding, with stirring, the mixture M1 obtained in step a) or the optional emulsion (E1) obtained in step a1) in a composition C3 to obtain an emulsion (E2) comprising either drops of the mixture M1 dispersed in composition C3, or drops of the emulsion (E1) dispersed in composition C3,
wherein the composition C3 comprises at least one photoinitiator and at least one type of monomers and/or oligomers, which are different from the at least one type of monomers and/or oligomers of composition C2, wherein the compositions C3 and C2 are not miscible with each other and wherein the composition C3 and the aqueous composition of step a1) are not miscible with each other,,
c) applying a shear to the emulsion (E2) obtained in step b), at a rate of between 100 s⁻¹ and 100,000 s⁻¹,
to obtain an emulsion (E3) comprising controlled-size drops and preferably monodisperse drops of the mixture M1 or the emulsion (E1) dispersed in the composition C3;
d) adding, with stirring, the emulsion (E3) obtained in step c) in a composition C4 to obtain an emulsion (E4) comprising drops of the emulsion (E3) dispersed in composition C4, wherein the compositions C3 and C4 are not miscible with each other,
the viscosity of composition C4 being greater than 10,000 mPa.s at 25° C at a shear rate of 10 s⁻¹ and being less than 10,000 mPa.s at 25° C at a shear rate of between 100 s⁻¹ and 100,000 s⁻¹; and
e) polymerizing of the compositions C2 and C3, to obtain solid microcapsules, dispersed in the composition C4.

11. The method according to claim **10,** wherein the monomers and/or oligomers of composition C2 have a functionality of at least 2, preferably of at least 5, more preferably of at least 10, even more preferably of at least 16.

12. The method according to claim **10** or **11,** wherein the at least one type of monomers and/or oligomers of composition C2 is selected from the group consisting of: 1,6-hexanediol diacrylate, 3-methyl-1,5-pentanediyl diacrylate, tripropylene glycol diacrylate, propylidynetrimethyl trimethacrylate, trimethylolpropane triacrylate, 1,10-decanediyl diacrylate, bisphenol A epoxy diacrylate, epoxidized soybean oil acrylate, polyester acrylate oligomer, dendritic acrylate, epoxy acrylate oligomer, urethane acrylates, polyester acrylate, epoxy acrylate, amine acrylate, oligoamine acrylate, silicon elastomers and combinations thereof.

13. The method according to any one of claims **10** to **12,** wherein the at least one active agent of composition C1 has a solubility in the composition C3 which is equal to or greater than 1 % w/w.

14. The method according to any one of claims **10** to **13,** wherein the mixture M1 comprises:
- from 45% to 99%, preferably from 50% to 70%, more preferably about 70%, of the at least one active agent,
- from 0.5% to 5%, preferably from 1.5% to 4%, more preferably from 1.5% to 3%, of the at least one initiator,
- from 0.5% to 50%, preferably from 5% to 15%, more preferably from 10% to 13%, of the at least one type of monomers and/or oligomers,
the percentages being expressed by weight relative to the total weight of the mixture M1.

15. The method according to any one of claims **10** to **13,** wherein the mixture M1 comprises:
- from 10% to 40%, preferably from 20% to 40%, more preferably about 30%, of the at least one active agent,
- from 0.5% to 5%, preferably from 1.5% to 4%, more preferably from 1.5% to 3%, of the at least one initiator,
- from 55% to 89.5%, preferably from 55% to 80%, more preferably from 55% to 65%, of the at least one type of monomers and/or oligomers,
the percentages being expressed by weight relative to the total weight of the mixture M1.

16. The method according to any one of claims **10** to **15,** wherein the at least one initiator of the composition C2 and/or the at least one photoinitiator of the composition C3 is selected from the group consisting of: 2-hydroxy-2-methylpropiophenone, phenyl(2,4,6-trimethylbenzoyl)phosphinate ethyl, bis(2,4,6-trimethylbenzoyl)phenylphosphine oxide and combinations thereof.

17. The method according to any one of claims **10** to **16,** wherein said method is for preparing solid microcapsules according to any one of claims **1** to **9.**
